# FASCICULE DE BREVET EUROPEEN

(11) **EP 3 016 728 B1**
(45) Date de publication et mention de la délivrance du brevet: **15.11.2017**
(21) Numéro de dépôt: 14733280.3
(22) Date de dépôt: 06.06.2014
(51) Int. Cl.: B01D 3/14, B01D 3/32, C07C 7/04, C10G 69/04, B01D 3/00, C10G 45/72, C10G 7/12, C10G 11/18

(54) **PROCÉDÉ DE CHAUFFAGE DE LA COLONNE DE DISTILLATION DE LA COUPE C3 ISSUE D'UNE UNITÉ FCC AU MOYEN D'UN CIRCUIT D'EAU CHAUFFÉE PAR DES FLUX APPARTENANT A DES UNITÉS PLACÉES EN AMONT ET/OU EN AVAL DE L'UNITÉ FCC**
VERFAHREN ZUM ERHITZEN DER DESTILLATIONSSÄULE DER C3-FRAKTION AUS EINER FCC-ANLAGE MITTELS EINER SCHALTUNG VON WASSER, ERHITZT DURCH STRÖME, DIE ZU DER FCC-ANLAGE VOR- UND/ODER NACHGESCHALTETEN EINHEITEN GEHÖREN
PROCESS FOR HEATING THE COLUMN FOR DISTILLATION OF THE C3 FRACTION FROM AN FCC UNIT BY MEANS OF A CIRCUIT OF WATER HEATED BY STREAMS BELONGING TO UNITS PLACED UPSTREAM AND/OR DOWNSTREAM OF THE FCC UNIT

(30) Priorité: 01.07.2013 FR 1356393
(43) Date de publication de la demande: 11.05.2016
(73) Titulaire: IFP Energies nouvelles, 92500 Rueil-Malmaison (FR)
(72) Inventeur: DIGNE, Romina, 69007 Lyon (FR); DREUX, Héloïse, 69007 Lyon (FR); FEUGNET, Frédéric, 69004 Lyon (FR); LAMBERT, Nicolas, 92130 Issy Les Moulineaux (FR)
(86) Numéro de dépôt international: PCT/FR2014/051359
(87) Numéro de publication internationale: WO 2015/001214

(56) Documents cités:
- EP-A1- 2 088 184
- US-A- 4 411 676

## Description

### DOMAINE DE L'INVENTION

L'invention se situe dans le domaine de la séparation par fractionnement du propane et du propylène issus d'une unité de craquage catalytique en lit fluidisé (FCC). Des coupes C3 provenant d'unité de cokéfaction retardée, de viscoréducteur ou de tout autre procédé visant à produire du propylène, peuvent également s'ajouter à la coupe C3 issu du FCC afin de séparer le propane du propylène présent dans ces coupes. La pureté du propylène obtenu après fractionnement est généralement celle correspondant au grade polymère (> 99,5% poids).

Le propane et le propylène ont des températures d'ébullition très proches, la séparation par fractionnement est donc très coûteuse en investissement et en utilités, et il existe donc un important besoin pour réduire cette dépense énergétique.

### EXAMEN DE L'ART ANTERIEUR

La séparation par fractionnement du propane et du propylène peut se faire typiquement :
- soit à haute pression (environ 20-25 bars) avec condensation des vapeurs de tête par de l'eau de refroidissement
- soit à faible pression (environ 10-15 bars et moins) avec recompression des vapeurs de tête pour rebouillir la colonne (schéma avec pompe à chaleur)

La séparation du propane et du propylène par fractionnement avec une pompe à chaleur est très souvent plus avantageuse économiquement, mais elle est plus compliquée à opérer. Certains raffineurs préfèrent donc utiliser une séparation "haute pression" avec condensation des vapeurs de tête par de l'eau de refroidissement.
Dans le cas d'un fractionnement "haute pression", la température en fond de colonne est environ 63°C. Le rebouillage, c'est à dire l'apport de calories nécessaires à atteindre la température en fond de colonne, peut se faire grâce à :
- des fluides procédés de l'unité FCC,
- de la vapeur basse pression,
- de l'eau chaude circulant dans une boucle fermée (circuit d'eau chaude).
Le document EP 2088184 décrit la récupération de la chaleur en tête d'une unité FCC pour le rebouillage de la colonne de fractionnement propane/propylène. Généralement, le rebouillage de la colonne de fractionnement propane/propylène ne se fait pas intégralement par échange thermique avec des fluides procédés de l'unité FCC pour les raisons suivantes :
- les besoins énergétiques sont très élevés,
- les fluides procédés à refroidir dont la température est supérieure à 200°C sont utilisés pour chauffer des flux plus chauds que le fond de la colonne de fractionnement propane/propylène ou pour produire de la vapeur moyenne et haute pression, afin d'avoir une intégration thermique plus efficace.

Les fluides procédés utilisés pour rebouillir la colonne de fractionnement propane/propylène sont généralement l'essence issue du débutaniseur et/ou le reflux circulant supérieur de la colonne de fractionnement principal, fluides ayant des températures modérées.

Un rebouillage, au moins partiel, de la colonne de fractionnement propane/propylène avec de la vapeur basse pression ou de l'eau chaude circulant dans une boucle fermée est donc généralement nécessaire.

Le principe du circuit d'eau chaude est le suivant :
- de l'eau à 65°C environ est chauffée à une température de 91°C environ au moyen de fluides procédés de l'unité FCC dans une configuration variant selon la puissance nécessaire au rebouillage de la colonne de fractionnement propane/propylène ainsi que de la disponibilité des flux suivants :
   ∘ vapeurs de tête de la colonne de fractionnement principal de l'unité FCC,
   ∘ vapeurs issues des différents étages du compresseur de gaz craqués (Wet Gas Compressor),
   ∘ essence issue du débutaniseur de l'unité FCC,
   ∘ reflux circulant supérieur de la colonne de fractionnement principal de l'unité FCC,
   ∘ vapeurs de tête de la colonne de séparation d'essence (naphta splitter), s'il y en a une, de l'unité FCC,
   ∘ LCO issu de la colonne de fractionnement principal de l'unité FCC.
- l'eau à 91°C environ, va rebouillir la colonne de fractionnement propane/propylène. Après avoir rebouilli la colonne, la température de l'eau est à nouveau égale à 65°C environ.
- l'eau à 65°C environ va de nouveau se faire chauffer jusqu'à 91°C environ au moyen de fluides procédés de l'unité FCC listés précédemment.

Le circuit d'eau chaude est un circuit en boucle fermée. Des pompes sont nécessaires pour la circulation de l'eau chaude dans ledit circuit, de même qu'un réservoir, un appoint d'eau pour compenser les pertes d'eau éventuelles et un aéroréfrigérant afin de dissiper la chaleur non utilisée durant les phases transitoires (démarrage, arrêt, etc.).

Le rebouillage de la colonne de séparation propane/propylène se fait typiquement à deux niveaux afin de bénéficier des meilleures approches thermiques possibles :
- avec un rebouilleur en fond de colonne utilisant l'eau chaude à 91°C environ et la renvoyant à 75°C environ,
- avec un rebouilleur intermédiaire utilisant l'eau chaude à 75°C environ et la renvoyant à 65°C environ.

Le rebouilleur intermédiaire correspond à environ 40% de la puissance totale des deux rebouilleurs. Le rebouilleur intermédiaire permet d'avoir une meilleure approche thermique sur l'ensemble des deux rebouilleurs.

La température de la tête de la colonne de fractionnement principal est généralement minimisée pour maximiser la qualité des produits issus du fractionnement et la récupération de chaleur tout en évitant la condensation d'eau dans la colonne de fractionnement pour des raisons de corrosion. La température en tête de colonne de fractionnement correspond généralement à la température de rosée de l'eau + 25°C.

La marge de 25°C permet d'éviter la corrosion dans la partie supérieure de la colonne. On a ainsi une température en tête de colonne de fractionnement généralement comprise entre 100°C et 120°C. Lorsqu'un circuit d'eau chaude est installé au sein de l'unité FCC, on est souvent amené à augmenter la température en tête de colonne de fractionnement pour avoir une meilleure approche thermique dans l'échangeur chauffant l'eau chaude. Cette pratique détériore la qualité du fractionnement et ne permet pas une optimisation thermique poussée.

De plus, lorsque l'unité FCC a un rendement en propylène élevé, ou lorsque la séparation de coupes riches en propylène extérieures à l'unité FCC est requise, le circuit d'eau chaude ne permet pas de rebouillir totalement la colonne de fractionnement propane/propylène.

Un rebouilleur avec de la vapeur basse pression est donc nécessaire pour compléter l'apport de calories.

### DESCRIPTION SOMMAIRE DES FIGURES

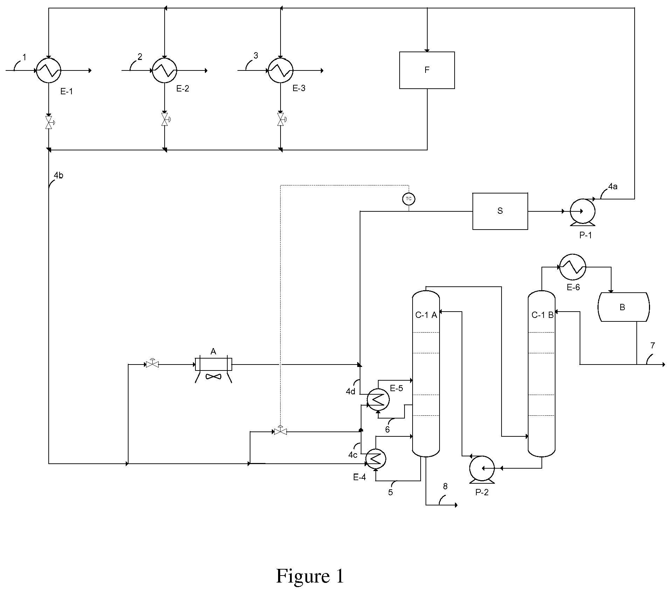

La figure 1 est un schéma du procédé de la boucle d'eau chaude selon la présente invention faisant apparaitre les différents fluides chauds possibles. La boucle elle-même présente autant d'échangeurs qu'il y a de fluides chauds nouveaux mis en oeuvre.

### DESCRIPTION SOMMAIRE DE L'INVENTION

La présente invention peut se définir comme un procédé de chauffage du rebouilleur du système de la colonne de séparation propane/propylène situé en aval d'une unité FCC et alimenté par la coupe C3 de ladite unité FCC. On utilise l'expression "système de colonne" pour indiquer que la séparation propane/propylène peut être réalisée par une ou plusieurs colonnes de séparation disposées en série.

Lorsqu'il y a une seule colonne, le rebouilleur est celui de la colonne considérée.

Lorsqu'il y a plusieurs colonnes disposées en série, le rebouilleur est celui de la première colonne au sens de l'écoulement des fluides, la tête de cette première colonne alimentant le fond de la seconde colonne et ainsi de suite s'il y a plus de deux colonnes.

Le procédé selon la présente invention consiste à chauffer l'eau d'un circuit d'eau chaude au moyen d'un ou plusieurs fluides procédés provenant d'unités placées en amont et/ou en aval de l'unité FCC et appelés fluides "chauds", lesdits fluides chauds étant choisis seuls ou en combinaison parmi les fluides suivants :
- vapeurs de tête de la colonne de fractionnement de l'unité d'hydrocraquage doux en amont de l'unité FCC,
- vapeurs de tête du stripeur de l'unité d'hydrocraquage doux en amont de l'unité FCC,
- effluent du (des) réacteur(s) d'hydrodésulfuration (HDS) de l'unité d'hydrotraitement d'essence FCC, s'il y en a une en aval de l'unité FCC, après échanges thermiques avec la charge du (des) réacteur(s) d'HDS et avec la charge du réacteur d'hydrogénation sélective (SHU).

Le chauffage de l'eau du circuit d'eau chaude selon la présente invention peut éventuellement être complété par l'utilisation d'au moins un des fluides suivants en plus d'un des fluides chauds :
- vapeurs de tête de la colonne de fractionnement principal de l'unité FCC,
- vapeurs issues des différents étages du compresseur de gaz craqués,
- essence issue du débutaniseur de l'unité FCC,
- reflux circulant supérieur de la colonne de fractionnement principal de l'unité FCC,
- vapeurs de tête de la colonne de séparation d'essence de l'unité FCC,
- LCO (light cycle oil) issu de la colonne de fractionnement principal de l'unité FCC.

Le procédé de chauffage du rebouilleur du système de colonne de séparation propane/propylène situé en aval d'une unité FCC selon la présente invention, est alimenté par la coupe C3 de ladite unité FCC à laquelle peut s'ajouter, selon une variante, une coupe C3 issue d'un autre procédé visant à produire du propylène. A titre d'exemple d'autre procédé (au sens distinct du FCC), on peut citer le procédé de conversion d'oléfines en propylène.

Le procédé de chauffage du rebouilleur de la colonne de séparation propane/propylène (C-1 A) selon la présente invention peut se définir plus précisément lorsque le système de séparation propane/propylène est constituée de deux colonnes C-1 A et C-1 B connectées en série de la façon suivante: le liquide en fond de colonne C-1 B est pompé puis envoyé en tête de colonne C-1 A, les vapeurs de tête de C-1 B sont condensées avec de l'eau de refroidissement puis envoyées dans le ballon de reflux B de la colonne C-1 B, le propylène (flux 7) est récupéré en tête de C-1 B et le propane (flux 8) en fond de C-1 A, les deux colonnes (C-1 A et C-1 B) étant situées en aval d'une unité FCC.

Le procédé selon l'invention se définit ainsi: l'eau du circuit d'eau chaude est chauffée de 65°C (flux 4a) à 91°C (flux 4b) via des échangeurs en parallèle (E-1, E-2, E-3 ...) par l'un au moins des nouveaux flux procédés suivants :
- vapeurs de tête de la colonne de fractionnement de l'unité d'hydrocraquage doux en amont de l'unité FCC (flux 1) au moyen de l'échangeur E-1,
- vapeurs de tête du stripeur de l'unité d'hydrocraquage doux en amont de l'unité FCC (flux 2) au moyen de l'échangeur E-2,
- effluent du (des) réacteur(s) d'HDS de l'unité d'hydrotraitement d'essence FCC (flux 3) au moyen de l'échangeur E-3, s'il y a une unité d'hydrotraitement d'essence FCC en aval de l'unité FCC. Le flux 3 ayant au préalable été utilisé pour chauffer la charge du (des) réacteur(s) d'HDS, et la charge du réacteur d'hydrogénation sélective (SHU),
le rebouillage de la colonne de séparation propane/propylène (C-1 A) se faisant à deux niveaux :
- avec un rebouilleur en fond de colonne (E-4), chauffant le flux 5 au moyen de l'eau chaude à 91°C (flux 4b) et renvoyant l'eau à 75°C environ (flux 4c),
- avec un rebouilleur intermédiaire (E-5), chauffant le flux 6 au moyen de l'eau chaude à 75°C environ (flux 4c) et renvoyant l'eau à 65°C (flux 4d).

### DESCRIPTION DETAILLEE DE L'INVENTION

La présente invention se situe dans le cadre de l'apport de calories au rebouilleur de la colonne de fractionnement propane/propylène située en aval de l'unité de craquage catalytique (notée en abrégé FCC).

L'invention consiste à chauffer l'eau d'un circuit d'eau chaude avec des fluides procédés d'unités placées en amont et/ou en aval de l'unité FCC, en complément éventuellement des fluides procédés habituels de l'unité FCC déjà décrits dans l'art antérieur.

Le circuit d'eau chaude permettant l'apport de calories au rebouilleur de la colonne de fractionnement propane/propylène est décrit dans la figure 1 selon l'invention.

Sur la figure 1 est représentée la colonne de fractionnement "haute pression" propane/propylène, généralement constituée de 2 colonnes (C-1 A et C-1 B) étant donné le nombre élevé de plateaux (entre 200 et 300) et les contraintes usuelles de hauteur liées à l'implantation de colonnes industrielles (environ 100 mètres au maximum).

Le liquide en fond de colonne C-1 B est pompé puis envoyé en tête de colonne C-1 A.

Les vapeurs de tête de C-1 B sont condensées avec de l'eau de refroidissement puis envoyées dans le ballon de reflux B.

Le propylène (flux 7) est récupéré en tête de C-1 B et le propane (flux 8) en fond de C-1 A.

Dans le procédé selon l'invention, l'eau du circuit d'eau chaude est chauffée de 65°C (flux 4a) à 91°C (flux 4b) via des échangeurs en parallèle (E-1, E-2, E-3...) par les nouveaux flux procédés suivants :
- vapeurs de tête de la colonne de fractionnement de l'unité d'hydrocraquage doux en amont de l'unité FCC (flux 1) au moyen de l'échangeur E-1,
- vapeurs de tête du stripeur de l'unité d'hydrocraquage doux en amont de l'unité FCC (flux 2) au moyen de l'échangeur E-2,
- effluent du (des) réacteur(s) d'HDS de l'unité d'hydrotraitement d'essence FCC (flux 3) au moyen de l'échangeur E-3, s'il y a une unité d'hydrotraitement d'essence FCC en aval de l'unité FCC. Le flux 3 ayant au préalable été utilisé pour chauffer la charge du (des) réacteur(s) d'HDS, et la charge du réacteur d'hydrogénation sélective (SHU).

Les fluides du circuit d'eau chaude peuvent également être complétés par les fluides procédés de l'unité FCC décrits dans l'art antérieur via l'opération F.

Le rebouillage de la colonne de séparation propane/propylène (C-1 A) se fait à deux niveaux :
- avec un rebouilleur en fond de colonne (E-4), chauffant le flux 5 au moyen de l'eau chaude à 91°C (flux 4b), et renvoyant l'eau à 75°C environ (flux 4c),
- avec un rebouilleur intermédiaire (E-5), chauffant le flux 6 au moyen de l'eau chaude à 75°C environ (flux 4c) et renvoyant l'eau à 65°C (flux 4d).

Dans le procédé selon l'invention, il est possible de chauffer davantage d'eau chaude et ainsi obtenir un rebouillage de la colonne de fractionnement propane/propylène sans consommer de la vapeur basse pression.

De plus, les fluides procédés des unités en amont et en aval de l'unité FCC, permettant de chauffer l'eau, ont une température généralement plus élevée que les fluides procédés de l'unité FCC (Tableau 1 ci-dessous).

Par conséquent, la différence de température entre les flux chauds et froids dans les échangeurs (E-1, E-2, E-3...) chauffant l'eau chaude est plus grande, ce qui réduit le coût desdits échangeurs, donc le coût du circuit d'eau chaude.

**Tableau 1 : Températures des fluides procédés pouvant chauffer l'eau du circuit d'eau chaude**

| | Flux procédé | Température (°C) |
|---|---|---|
| Nouveaux flux selon l'invention | Vapeurs de tête de la colonne de fractionnement (un d'hydrocraquage doux) | 120 à 140 |
| | Vapeurs de tête du stripeur (unité d'hydrocraquage doux) | 150 à 180 |
| | Effluent du (des) réacteur(s) d'HDS (unité d'hydrotraitement d'essence FCC) | > 120 |
| Flux connus de l'art antérieur | Vapeurs de tête de la colonne de fractionnement principal (un FCC) | 100 à 120 |
| | Essence issue du débutaniseur (unité FCC) | 120 à 180 |
| | Reflux circulant supérieur de la colonne de fractionnement principal (unité FCC) | 120 à 140 |
| | Vapeurs de tête de la colonne de séparation d'essence (unité FC | 110 à 130 |
| | LCO issu de la colonne de fractionnement principal (unité FCC | > 160 |

Tous ces flux, nouveaux ou déjà connus de l'art antérieur, sont susceptibles de chauffer l'eau circulant dans la boucle d'eau chaude utilisée pour rebouillir la colonne de séparation propane/propylène selon une configuration variant selon la puissance nécessaire au rebouillage de la colonne de fractionnement propane/propylène ainsi que de la disponibilité des flux.

Le procédé selon la présente invention est mis en jeu dès qu'il fait appel soit à la vapeur de tête de la colonne de fractionnement liée à l'unité d'hydrocraquage doux, soit à la vapeur de tête du stripeur lié à l'unité d'hydrocraquage doux, soit encore à l'effluent chaud du ou des réacteurs d'HDS (lorsqu'une telle unité existe). Toute combinaison de ces trois flux, pris en partie ou dans leur intégralité, rentre dans le cadre de la présente invention.

Les flux ayant une température élevée seront privilégiés pour minimiser l'aire des échangeurs mis en oeuvre. Mais il doit être compris comme faisant partie de la présente invention toute combinaison de fluides (nouveaux ou faisant partie de l'art antérieur) dès qu'au moins un fluide nouveau parmi les trois précédents est utilisé dans la boucle d'eau chaude.

### EXEMPLE SELON L'INVENTION

Une unité d'hydrocraquage doux traitant 458 t/h de distillat sous-vide (79% provenant de fractionnement sous-vide et 21% provenant d'une unité de cokéfaction) produit 304 t/h de résidu.

La section réactionnelle de l'unité d'hydrocraquage doux comporte 3 réacteurs en série et 7 lits catalytiques. La température moyenne de chaque lit est de 403°C au cours de la durée de vie du catalyseur. La pression partielle en hydrogène est 85 bars abs et la vitesse spatiale horaire (VVH) est 0,31 h⁻¹.

Le résidu de l'unité d'hydrocraquage doux est envoyé dans une unité FCC opérant dans des conditions sévères et avec ajout de ZSM-5 dans le catalyseur pour maximiser le rendement en propylène.

Le rendement en propylène de l'unité FCC est de 9% poids par rapport à la charge.

L'unité FCC comprend une section de fractionnement des GPL (gaz de pétrole liquéfiés) constituée d'un dépropaniseur, d'un dééthaniseur et d'une colonne de fractionnement propane/propylène haute pression pour obtenir du propylène avec une pureté de 99,6% mol.

Une coupe C3 provenant d'une unité de cokefaction (13 t/h) est ajoutée aux GPL issus du FCC en entrée de la section de fractionnement des GPL.

La colonne de séparation propane/propylène a une charge de 47 t/h dont la composition massique est la suivante : 66% de propylène, 33% de propane et moins de 1% de C4+.

La pression en fond de colonne de fractionnement propane/propylène est 22 bars abs.

La puissance thermique nécessaire pour le rebouillage de la colonne de séparation propane/propylène est de 52 MW (MW est l'abréviation de mega watt soit 10⁶ watt).

Il n'y a pas d'unité d'hydrotraitement d'essence FCC en aval de l'unité FCC car la teneur en soufre de l'essence issue de l'unité FCC est inférieure à 10 ppm.

Les fluides procédés de l'unité FCC disponibles pour chauffer l'eau du circuit d'eau chaude sont indiqués dans le Tableau 2 ci-dessous.

La différence de température en moyenne logarithmique (DTLM) est indiquée pour chaque échange. Plus la valeur DTLM est grande, plus la surface d'échange sera faible.

**Tableau 2 : Flux disponibles pour chauffer l'eau du circuit d'eau chaude (selon l'art antérieur)**

| Fluides procédés de l'unité FCC disponibles | T entrée (°C) | T sortie (°C) | Duty (MW) | DTLM (°C) |
|---|---|---|---|---|
| Vapeurs de tête de la colonne de fractionnement principal | 100 | 70 | 20 | 6,8 |
| Essence issue du débutaniseur | 158 | 70 | 11 | 23,9 |
| Total | | | 31 | |

Seuls 31 MW sur les 52 MW nécessaires peuvent être fournis en interne de l'unité FCC.
Dans cet exemple :
- il n'y a pas de colonne de séparation d'essence,
- l'essence en fond de débutaniseur chauffe d'abord la charge du débutaniseur puis l'eau chaude,
- le reflux circulant supérieur du fractionnement principal ne peut être utilisé, car il chauffe le rebouilleur du dééthaniseur,
- le LCO ne peut être utilisé car il chauffe la charge du débutaniseur.

Au total, 21 MW de vapeur basse pression sont donc nécessaires pour compléter la chauffe du rebouilleur de la colonne de fractionnement propane/propylène.

Le Tableau 3 ci-dessous indique les fluides procédés disponibles pour chauffer l'eau du circuit d'eau chaude selon l'invention.

**Tableau 3 : Flux disponibles pour chauffer l'eau du circuit d'eau chaude (selon l'invention)**

| | Fluides procédés disponibles (unité) | T entrée (°C) | T sortie (°C) | Duty (MW) | DTLM (°C) |
|---|---|---|---|---|---|
| Flux selon l'art antérieur | Vapeurs de tête de la colonne fractionnement principal (unité FCC) | 100 | 70 | 20 | 6,8 |
| | Essence issue du débutaniseur (unité FCC) | 158 | 70 | 11 | 23,9 |
| Nouveaux flux selon l'invention | Vapeurs de tête de la colonne fractionnement (unité d'hydrocraquage dou | 121 | 88 | 34 | 26,3 |
| | Vapeurs de tête du stripeur (unité d'hydrocraquage doux) | 150 | 70 | 8 | 21,9 |
| **Total** | | | | **73** | |

Le Tableau 3 montre que la puissance thermique disponible pour chauffer l'eau (73 MW) est supérieure aux besoins (52 MW). Les échanges avec les différences de température (DTLM) les plus élevées sont préférés car ils sont moins coûteux. Dans cet exemple, la colonne de séparation propane/propylène peut être rebouillie totalement par un circuit d'eau chaude comprenant les échanges suivants pour chauffer l'eau :
- vapeurs de tête de la colonne de fractionnement de l'unité d'hydrocraquage doux / eau chaude (34 MW),
- essence issue du débutaniseur / eau chaude (11 MW),
- vapeurs de tête du stripeur de l'unité d'hydrocraquage doux / eau chaude (8 MW).

L'eau chaude n'a plus besoin d'être chauffée par les vapeurs de tête de la colonne de fractionnement principal de l'unité FCC. Cet échange a été supprimé car il présentait la plus faible différence de température (DTLM) et donc le ratio "surface d'échange/puissance échangée" le plus élevé.

## Revendications

1. Procédé de chauffage du rebouilleur du système de colonne de séparation propane/propylène (C-1 A, C-1 B) situé en aval d'une unité FCC et alimenté par la coupe C3 de ladite unité FCC, procédé consistant à chauffer l'eau d'un circuit d'eau chaude au moyen d'un ou plusieurs fluides procédés provenant d'unités placées en amont et/ou en aval de l'unité FCC et appelés fluides chauds, lesdits fluides chauds étant choisis seuls ou en combinaison parmi les fluides suivants :
- vapeurs de tête de la colonne de fractionnement de l'unité d'hydrocraquage doux (1) en amont de l'unité FCC,
- vapeurs de tête du stripeur de l'unité d'hydrocraquage doux (2) en amont de l'unité FCC,
- effluent du (des) réacteur(s) d'HDS de l'unité d'hydrotraitement d'essence FCC (3) placée en aval de l'unité FCC, après échanges thermiques avec la charge du (des) réacteur(s) d'HDS et avec la charge du réacteur d'hydrogénation sélective (SHU).

2. Procédé de chauffage du rebouilleur du système de colonne de séparation propane/propylène situé en aval d'une unité FCC selon la revendication 1, dans lequel au moins un fluide parmi les fluides suivants chauffe également le circuit d'eau chaude en plus d'un des fluides chauds :
- vapeurs de tête de la colonne de fractionnement principal de l'unité FCC,
- vapeurs issues des différents étages du compresseur de gaz craqués,
- essence issue du débutaniseur de l'unité FCC,
- reflux circulant supérieur de la colonne de fractionnement principal de l'unité FCC,
- vapeurs de tête de la colonne de séparation d'essence de l'unité FCC,
- LCO issu de la colonne de fractionnement principal de l'unité FCC.

3. Procédé de chauffage du rebouilleur du système de colonne de séparation propane/propylène situé en aval d'une unité FCC selon la revendication 1, alimenté par la coupe C3 de ladite unité FCC, ainsi que par une coupe C3 issue d'un autre procédé visant à produire du propylène.

4. Procédé de chauffage du rebouilleur du système de colonne de séparation propane/propylène situé en aval d'une unité FCC selon la revendication 3, dans lequel la coupe C3 issue d'un autre procédé que le FCC, provient d'une unité de conversion d'oléfines en propylène.

## Patentansprüche

1. Verfahren zum Erhitzen des Verdampfers des Propan/Propylen-Trennkolonnensystems (C-1 A, C-1 B), das einer FCC-Einheit nachgeschaltet ist und dem der C3-Schnitt dieser FCC-Einheit zugeführt wird, wobei das Verfahren im Erhitzen des Wassers eines Heißwasserkreislaufs mithilfe eines oder mehrerer Verfahrensflüssigkeiten besteht, die aus Einheiten stammen, die der FCC-Einheit vorgeschaltet und/oder nachgeschaltet sind und Heißflüssigkeiten genannt werden, wobei diese Heißflüssigkeiten allein oder in Kombination aus den folgenden Flüssigkeiten ausgewählt sind:
- Kopfdämpfe der Fraktionierungskolonne der Einheit zum milden Hydrocracken (1), die der FCC-Einheit vorgeschaltet ist,
- Kopfdämpfe des Strippers der Einheit zum milden Hydrocracken (2), die der FCC-Einheit vorgeschaltet ist,
- dem Abfluss des HDS-Reaktors (der HDS-Reaktoren) der Einheit zur Hydrobehandlung von FCC-Benzin (3), die der FCC-Einheit nachgeschaltet ist, nach Wärmeaustausch mit dem Einsatz des HDS-Reaktors (der HDS-Reaktoren) und mit dem Einsatz des Reaktors zur selektiven Hydrierung (SHU).

2. Verfahren zum Erhitzen des Verdampfers des Propan/Propylen-Trennkolonnensystems, das einer FCC-Einheit nachgeschaltet ist, gemäß Anspruch 1, wobei mindestens eine Flüssigkeit aus den folgenden Flüssigkeiten, neben einer der Heißflüssigkeiten auch den Heißwasserkreislauf erhitzt:
- Kopfdämpfe der Hauptfraktionierungskolonne der FCC-Einheit,
- Dämpfe, die aus verschiedenen Stufen des Verdichters der gecrackten Gase stammen,
- Benzin, das aus dem Entbutanisierer der FCC-Einheit stammt,
- oberer zirkulierender Rücklauf der Hauptfraktionierungskolonne der FCC-Einheit,
- Kopfdämpfe der Benzintrennkolonne der FCC-Einheit,
- LCO aus der Hauptfraktionierungskolonne der FCC-Einheit.

3. Verfahren zum Erhitzen des Verdampfers des Propan/Propylen-Trennkolonnensystems, das einer FCC-Einheit nachgeschaltet ist, nach Anspruch 1, dem der C3-Schnitt dieser FCC-Einheit zugeführt wird, sowie ein C3-Schnitt, der aus einem anderen Verfahren stammt, das zur Propylenproduktion bestimmt ist.

4. Verfahren zum Erhitzen des Verdampfers des Propan/Propylen-Trennkolonnensystems, das einer FCC-Einheit nachgeschaltet ist, nach Anspruch 3, wobei der C3-Schnitt, der aus einem anderen Verfahren stammt, aus einer Einheit zur Umwandlung von Olefinen in Propylen kommt.

## Claims

1. A process for heating the reboiler of the propane/propylene separation column system (C-1 A, C-1 B) disposed downstream of an FCC unit and fed with the C3 cut from said FCC unit, said process comprising heating the water in a hot water circuit by means of one or more process fluids coming from units disposed upstream and/or downstream of the FCC unit and referred to as hot fluids, said hot fluids being selected alone or in combination from the following fluids:
- overhead vapours from the fractionating column of the mild hydrocracking unit (1) upstream of the FCC unit,
- overhead vapours from the stripper of the mild hydrocracking unit (2) upstream of the FCC unit, and
- effluent from the HDS reactor or reactors of the FCC petrol hydrotreatment unit (3) disposed downstream of the FCC unit, after heat exchanges with the feedstock of the HDS reactor or reactors and with the feedstock of the selective hydrogenation reactor (SHU).

2. Process for heating the reboiler of the propane/propylene separation column system disposed downstream of an FCC unit according to claim 1 wherein at least one fluid from the following fluids also heats the hot water circuit in addition to one of the hot fluids:
- overhead vapours from the main fractionating column of the FCC unit,
- vapours from the different stages of the cracked gas compressor,
- petrol from the debutaniser of the FCC unit,
- upper circulating reflux from the main fractionating column of the FCC unit,
- overhead vapours from the petrol separation column of the FCC unit, and
- LCO from the main fractionating column of the FCC unit.

3. Process for heating the reboiler of the propane/propylene separation column system disposed downstream of an FCC unit according to claim 1 fed with the C3 cut of said FCC unit, as well as with a C3 cut from another process aiming to produce propylene.

4. Process for heating the reboiler of the propane/propylene separation column system disposed downstream of an FCC unit according to claim 3 wherein the C3 cut from another process than the FCC comes from a unit for the conversion of olefins into propylene.
